# EUROPEAN PATENT SPECIFICATION

(11) **EP 0 654 523 B1**
(45) Date of publication and mention of the grant of the patent: **04.08.1999**
(21) Application number: 94308426.9
(22) Date of filing: 15.11.1994
(51) Int. Cl.: C10G 11/18, C10G 57/00

(54) **Process for producing olefin(s)**
Verfahren zur Herstellung von Olefinen
Procédé pour la préparation d'oléfines

(30) Priority: 19.11.1993 US 154833
(43) Date of publication of application: 24.05.1995
(73) Proprietor: Exxon Research and Engineering Company, (a Delaware corp.), Florham Park, New Jersey 07932 (US)
(72) Inventor: Bearden Jr., Roby, Baton Rouge, Louisiana 70808 (US); Kerby, Michael Charles, Baton Rouge, Louisiana 70810 (US); Davis, Stephen Mark, Baton Rouge, Louisiana 70817 (US)
(74) Representative: Somers, Harold Arnold

(56) References cited:
- EP-A- 0 168 185
- EP-A- 0 259 156
- EP-A- 0 325 437
- EP-A- 0 577 280
- US-A- 3 894 935
- US-A- 4 968 401

## Description

This invention relates to a process for producing olefin(s).

The emergence of low emissions fuels has created a need to increase the availability of olefins for use in alkylation, oligomerization, MTBE and ETBE synthesis. In addition, a low cost supply of olefins continues to be in demand to serve as feedstock for polyolefin production.

Fixed bed processes for light paraffin dehydrogenation have recently attracted renewed interest for increasing olefin production. However, these type of processes typically require a high capital investment as well as a high operating cost. It is, therefore, advantageous to increase olefin yield using processes which require only a minimal amount of capital investment.

US-A-4,830,728 discloses a fluid catalytic cracking (FCC) unit which is operated to maximize olefin production. The FCC unit has two separate risers in which different feed streams are introduced. The operation of the risers is designed so that a certain catalyst will act to convert a heavy gas oil in one riser and a different catalyst will act to crack a lighter olefin/naphtha feed in the other riser. Conditions within the heavy gas oil riser are modified to maximize either gasoline or olefin production. The primary means of maximizing production of the desired product is by using a specified catalyst.

A problem inherent in producing olefin products using FCC units is that the process depends upon a specific catalyst balance to maximize production. In addition, even if a specific catalyst balance can be maintained to maximize overall olefin production. olefin selectivity is generally low due to undesirable side reactions such as extensive cracking, isomerization, aromatization and hydrogen transfer reactions. It is, therefore, desirable that olefin production be maximized in a process which allows a high degree of control over olefin selectivity.

EP-A-0325437 describes and claims a process for regenerating a coke-contaminated fluid cracking catalyst in a regeneration zone at a pressure in the range from above 240 kPa to 446 kPa and a temperature in the range from 650°C to 815°C while injecting the regeneration zone with enough oxygen-containing regeneration gas to maintain a dense fluid bed of regeneration catalyst, and regenerate the catalyst before returning it to a fluid cracker, comprising,
a) withdrawing a controlled stream of the regenerator catalyst and introducing it into a dehydrogenation zone at a temperature below those prevailing in the regeneration zone, the dehydrogenation zone being located in a catalyst cooler, externally relative to the cracker and regenerator, the amount of the stream being sufficient to supply the endothermic heat of reaction for dehydrogenation of alkanes in the dehydrogenation zone,
b) introducing a feedstream of the alkanes into the dehydrogenation zone in an amount sufficient to maintain hot withdrawn catalyst in a state of fluidization in the catalyst cooler, the state of fluidization existing in a sub-transport regime while maintained at a temperature high enough to convert at least 50% of the alkanes, and concurrently to cool the catalyst,
c) transporting the cooled catalyst from the dehydrogenation zone, the catalyst now at a temperature in the range from 650 to 731°C, to the regeneration zone, and mixing hot catalyst therein with the cooled catalyst; and
d) withdrawing products of dehydrogenation in an effluent stream from the catalyst cooler.

In one embodiment, the process comprises withdrawing a controlled stream of spent catalyst from the fluid cracker and introducing the spent catalyst directly into the dehydrogenation zone, and transporting the cooled catalyst for flow-controlled introduction into a riser of the fluid cracker, in the lower portion thereof, and in addition, introducing a minor amount relative to the alkanes, of steam into the dehydrogenation zone, the amount being sufficient, in combination with the alkanes to strip hydrocarbons remaining in the spent catalyst.

### SUMMARY OF THE INVENTION

In order to overcome problems inherent in the prior art, the present invention provides a process for producing olefin(s) from a feed comprising one or more C₂-C₁₀ alkanes, wherein the feed is contacted with an alkane-dehydrogenating catalyst consisting solely of coked catalytic cracking catalyst at alkane-dehydrogenating conditions to produce olefin(s), and recovering the resulting olefin(s)-containing product from the coked catalytic cracking catalyst.

In a preferred embodiment, the coked catalytic cracking catalyst has a carbon content of about 0.2-10 wt. %. More preferably, the coked catalytic cracking catalyst has a carbon content of about 0.3-5.0 wt.%.

In another preferred embodiment, the coked catalytic cracking catalyst is formed by contacting a coke precursor with a catalytic cracking catalyst. Preferably, the coke precursor is selected from the group consisting of light olefins, light and heavy naphthas, petroleum residuum, refinery sludge, tank bottoms, gas oils and torch oils.

The C₂-C₁₀ alkane feed stream used in another preferred embodiment of this invention is selected from the group consisting of ethane, propane, butane, pentane, hexane, heptane, octane, nonane, decane, isobutane, isopentanes, isohexanes, isoheptanes and iso-octanes. Preferably, the C₂-C₁₀ alkane feed stream is dehydrogenated at a temperature ofabout 800-1600°F, and the catalytic cracking catalyst which is used comprises a crystalline tetrahedral framework oxide component.

### DETAILED DESCRIPTION OF THE INVENTION

It has been found that a coked catalytic cracking catalyst can be used to enhance the dehydrogenation of an alkane feed stream to produce an olefin stream. This type of olefin production is advantageous since the olefin product can be used as a feedstock in other reaction processes to either increase the octane pool in a refinery or the olefins can be used in the manufacture of gasoline additives which are required to reduce undesirable hydrocarbon emissions.

The alkane feed stream of this invention is preferably a C₂-C₁₀ alkane composition. The alkane composition can be either branched or unbranched. Such compositions include ethane, propane, butane, pentane, hexane, heptane, octane, nonane, decane, isobutane, isopentanes, isohexanes, isoheptanes and iso-octanes.

The catalyst which is used in this invention can be any catalyst which is typically used to catalytically "crack" hydrocarbon feeds. Catalytic cracking is a process which is well known in the art of petroleum refining and generally refers to converting a large hydrocarbon molecule to a smaller hydrocarbon molecule by breaking at least one carbon to carbon bond. For example, large paraffin molecules can be cracked to a paraffin and an olefin, and a large olefin molecule can be cracked to two or more smaller olefin molecules. Long side chain molecules which may be present on aromatic rings or naphthenic rings can also be cracked.

It is preferred that the catalytic cracking catalyst that is used in this invention comprise a crystalline tetrahedral framework oxide component. This component is used to catalyze the breakdown of primary products from the catalytic cracking reaction into clean products such as naphtha for fuels and olefins for chemical feedstocks. Preferably, the crystalline tetrahedral framework oxide component is selected from the group consisting of zeolites, tectosilicates, tetrahedral aluminophophates (ALPOs) and tetrahedral silicoaluminophosphates (SAPOs). More preferably, the crystalline framework oxide component is a zeolite.

Zeolites which can be employed in accordance with this invention include both natural and synthetic zeolites. These zeolites include gmelinite, chabazite, dachiardite, clinoptilolite, faujasite, heulandite, analcite, levynite, erionite, sodalite, cancrinite, nepheline, lazurite, scolecite, natrolite, offretite, mesolite, mordenite, brewsterite, and ferrierite. Included among the synthetic zeolites are zeolites X, Y, A, L, ZK-4, ZK-5, B, E, F, H, J, M, Q, T, W, Z, alpha and beta, ZSM-types and omega.

In general, aluminosilicate zeolites are effectively used in this invention. However, the aluminum as well as the silicon component can be substituted for other framework components. For example, the aluminum portion can be replaced by boron, gallium, titanium or trivalent metal compositions which are heavier than aluminum. Germanium can be used to replace the silicon portion.

The catalytic cracking catalyst used in this invention can further comprise an active porous inorganic oxide catalyst framework component and an inert catalyst framework component. Preferably, each component of the catalyst is held together by attachment with an inorganic oxide matrix component.

The active porous inorganic oxide catalyst framework component catalyzes the formation of primary products by cracking hydrocarbon molecules that are too large to fit inside the tetrahedral framework oxide component. The active porous inorganic oxide catalyst framework component of this invention is preferably a porous inorganic oxide that cracks a relatively large amount of hydrocarbons into lower molecular weight hydrocarbons as compared to an acceptable thermal blank. A low surface area silica (e.g., quartz) is one type of acceptable thermal blank.

The inert catalyst framework component densifies, strengthens and acts as a protective thermal sink. The inert catalyst framework component used in this invention preferably has a cracking activity that is not significantly greater than the acceptable thermal blank. Kaolin and other clays as well as α-alumina, titania, zirconia, quartz and silica are examples of preferred inert components.

The inorganic oxide matrix component binds the catalyst components together so that the catalyst product is hard enough to survive interparticle and reactor wall collisions. The inorganic oxide matrix can be made from an inorganic oxide sol or gel which is dried to "glue" the catalyst components together. Preferably, the inorganic oxide matrix will be comprised of oxides of silicon and aluminum. It is also preferred that separate alumina phases be incorporated into the inorganic oxide matrix. Species of aluminum oxyhydroxides-γ-alumina, boehmite, diaspore, and transitional aluminas such as α-alumina, β-alumina, γ-alumina, δ-alumina, ε-alumina, κ-alumina, and ρ-alumina can be employed. Preferably, the alumina species is an aluminum trihydroxide such as gibbsite, bayerite, nordstrandite, or doyelite.

According to this invention, in order to produce an olefin stream, an olefin forming reaction is commenced by contacting an alkane feed stream with a coked catalytic cracking catalyst. The coked catalytic cracking catalyst is a catalytic cracking catalyst which contains a measurable content of carbonaceous material (i.e., coke) on the catalyst, and which will effectively enhance dehydrogenation of the alkane feed stream to selectively form an olefin product. Preferably, the carbon content of the coked catalytic cracking catalyst will be about 0.2-10 wt %, more preferably from about 0.3-5.0 wt. %, and most preferably about 0.4-2.5 wt.%.

A coked catalytic cracking catalyst can be obtained by any of numerous means. Such means are the subject-matter of EP-A-0 654 519, EP-A-0 654 520, EP-A-0 654 521 and EP-A-0 654 522, all having the same filing date. As one example, the coked catalytic cracking catalyst can be obtained as a result of a partial or incomplete regeneration of at least a portion of the spent catalyst stream in a FCC unit (EP-A-0 654 522). One of ordinary skill in the art will be able to attain the desired concentration of coke on the catalytic cracking catalyst using well known means of adjusting temperature, oxygen content or burn time within the regenerator portion of the FCC unit.

In another embodiment, fresh or fully regenerated catalytic cracking catalyst can be used by applying a precoking additive. Preferably, the precoking additive is added to a catalytic cracking catalyst after the catalyst has been fully regenerated in the regenerator portion of the FCC unit. Materials which can be used as a precoking additive are compounds which effectively form carbonaceous deposits on the catalyst surface. Examples of these compounds include light olefins, light and heavy naphthas, petroleum residuum, refinery sludge, tank bottoms, gas oils, FCC cycle oils and bottoms, and torch oils.

The amount of precoking additive that will be used to coke the catalytic cracking catalyst will be highly dependent upon the amount of carbon material that may be present on the catalytic cracking catalyst. The more carbon material that is already on the catalytic cracking catalyst, the less that will be needed to coke the catalyst to the desired level. The initial coke content should, therefore, be measured to determine if a precoking additive is needed. Methods of determining coke content are well known to those of ordinary skill in the art. Once the initial coke content is determined, the corresponding amount of coke precursor is added to achieve the desired final coke content.

The conversion of alkane to olefin in this invention generally involves a dehydrogenation reaction. In the dehydrogenation reaction, alkanes are converted to olefins and molecular hydrogen. This reaction is highly endothermic. Preferably, the reaction is carried out at a temperature of about 800-1600°F (426.7 to 871°C), more preferably about 800-1400°F (426.7 to 760°C).

The olefin producing reaction is somewhat dependent upon pressure. In general, the higher the pressure, the lower the conversion of alkane to olefin. Preferably, the process is carried out at about 0-100 psig (1.014 to 7.910 bar).

The contact time between the alkane stream and the coked catalytic cracking catalyst will also affect the yield of olefin product. Typically, optimal contact between the coked catalyst and the alkane stream is attained when the olefin product stream contains a concentration of at least about 1 wt % total olefin. Preferably, alkane vapor residence time will range from about 0.5-60 seconds, more preferably, about 1.0-10 seconds.

The invention will be further understood by reference to the following Example, which includes a preferred embodiment of the invention.

### EXAMPLE

An equilibrium zeolite beta FCC catalyst (SiO₂ 65.1 wt %; Al₂O₃ wt %; Na₂O 0.28 wt %; REO₂ 2.14 wt %) was placed in a fixed bed quartz reactor. The temperature of the reactor was maintained at 1250°F, and the pressure was maintained at 0 psig. Six runs were made varying the carbon content on the catalyst from 0.2 wt % (no pretreatment) to 2.7 wt% (pretreatment with either heavy cat naphtha (HCN) or petroleum residuum (resid)). Iso-butane feed was passed through the reactor at 1 second residence time and GHSV of 1066. The results are shown in Table 1.

**Table 1**

| Run Number | 001 | 002 | 003 | 004 | 005 | 006 |
|---|---|---|---|---|---|---|
| Feed Pre-Treat | none | HCN | HCN | Resid | Resid | Resid |
| Cat/Oil Pre-Treat | --- | 5.1 | 3.0 | 4.8 | 3.0 | 1.8 |
| Carbon Content (wt%) | 0.2 | 0.8 | 1.1 | 2.2 | 2.5 | 2.7 |
| | | | | | | |
| Feed | i-C₄H₁₀ | i-C₄H₁₀ | i-C₄H₁₀ | i-C₄H₁₀ | i-C₄H₁₀ | i-C₄H₁₀ |
| | | | | | | |
| Iso-C₄H₁₀ Conversion (wt%) | 45.3 | 37.8 | 39.4 | 33.1 | 34.3 | 36.0 |
| | | | | | | |
| Selectivity (%) | | | | | | |
| C₁-C₃ | 55.1 | 43.8 | 41.7 | 35.0 | 35.6 | 36.2 |
| n-C₄H₁₀ | 3.0 | 0.3 | 2.2 | 1.8 | 1.8 | 2.0 |
| 1-C₄H₈ | 5.6 | 7.0 | 6.3 | 5.6 | 5.8 | 5.8 |
| t-2-C₄H₈ | 5.9 | 6.9 | 6.3 | 5.6 | 5.6 | 5.8 |
| c-2-C₄H₈ | 5.3 | 5.6 | 5.1 | 4.5 | 4.6 | 4.6 |
| Iso-C₄H₈ | 20.8 | 31.1 | 36.4 | 45.5 | 45.1 | 44.0 |
| >C4's | 4.4 | 5.5 | 2.1 | 1.4 | 1.5 | 1.6 |
| | | | | | | |
| Iso-C₄H₈ Yield (wt%) | 9.4 | 11.7 | 14.3 | 15.0 | 15.5 | 15.8 |

Having now fully described this invention, it will be appreciated by those skilled in the art that the invention can be performed within a wide range of parameters within what is claimed.

## Claims

1. A process for producing olefin(s) from a feed comprising one or more C₂-C₁₀ alkanes, wherein the feed is contacted with an alkane-dehydrogenating catalyst consisting solely of coked catalytic cracking catalyst at alkane-dehydrogenating conditions to produce olefin(s), and recovering the resulting olefin(s)-containing product from the coked catalytic cracking catalyst.

2. The process of claim 1, wherein the coked catalytic cracking catalyst has a carbon content in a range of from 0.2 to 10 wt. %.

3. The process of claim 2, wherein the coked catalytic cracking catalyst has a carbon content in a range of from 0.3 to 5.0 wt. %.

4. The process of any preceding claim, wherein the coked catalytic cracking catalyst is formed by contacting a coke precursor with a catalytic cracking catalyst.

5. The process of claim 4, wherein the coke precursor is selected from light olefins, light and heavy naphthas, petroleum residuum, refinery sludge, tank bottoms, gas oils, FCC cycle oils and bottoms, and torch oils.

6. The process of any preceding claim, wherein the feed is selected from one or more of ethane, propane, butane, pentane, hexane, heptane, octane, nonane, decane, isobutane, isopentanes, isohexanes, isoheptanes and iso-octanes.

7. The process of any preceding claim, wherein the dehydrogenation of the feed is conducted at a temperature in a range of from 800 to 1600°F (426 to 871°C).

8. The process of any preceding claim, wherein the catalytic cracking catalyst comprises a crystalline tetrahedral framework oxide component.

## Patentansprüche

1. Verfahren zur Herstellung von Olefin(en) aus einem Einsatzmaterial, das ein oder mehrere C₂- bis C₁₀-Alkane umfaßt, wobei das Einsatzmaterial mit einem Alkan-Dehydrierkatalysator, der nur aus verkoktem katalytischen Crackkatalysator besteht, bei Alkan-Dehydrierbedingungen kontaktiert wird, um Olefin(e) zu erzeugen, und das resultierende, Olefin(e) enthaltende Produkt von dem verkokten katalytischen Crackkatalysator gewonnen wird.

2. Verfahren nach Anspruch 1, bei dem der verkokte katalytische Crackkatalysator einen Kohlenstoffgehalt im Bereich von 0,2 bis 10 Gew.% hat.

3. Verfahren nach Anspruch 2, bei dem der verkokte katalytische Crackkatalysator einen Kohlenstoffgehalt im Bereich von 0,3 bis 5,0 Gew.% hat.

4. Verfahren nach einem der vorhergehenden Ansprüche, bei dem der verkokte katalytische Crackkatalysator gebildet wird, indem ein Koksvorläufer mit katalytischem Crackkatalysator kontaktiert wird.

5. Verfahren nach Anspruch 4, bei dem der Koksvorläufer ausgewählt ist aus leichten Olefinen, leichten und schweren Naphthas, Erdölrückständen, Raffinerieschlamm, Tank-Bodenprodukt, Gasölen, FCC-Cyclusölen und Bodenprodukten, und Fackelölen.

6. Verfahren nach einem der vorhergehenden Ansprüche, bei dem das Einsatzmaterial ausgewählt ist aus einem oder mehreren von Ethan, Propan, Butan, Pentan, Hexan, Heptan, Octan, Nonan, Decan, Isobutan, Isopentanen, Isohexanen, Isoheptanen und Isooctanen.

7. Verfahren nach einem der vorhergehenden Ansprüche, bei dem die Dehydrierung des Einsatzmaterials bei einer Temperatur im Bereich von 426°C bis 871°C (800 bis 1600°F) durchgeführt wird.

8. Verfahren nach einem der vorhergehenden Ansprüche, bei dem der katalytische Crackkatalysator eine kristalline tetraedrische Grundgerüstoxidkomponente umfaßt.

## Revendications

1. Procédé de production d'oléfine(s) à partir d'une charge d'alimentation comprenant un ou plusieurs alcanes en C₂-C₁₀, dans lequel la charge d'alimentation est mise en contact avec un catalyseur de déshydrogénation d'alcanes constitué uniquement d'un catalyseur de craquage catalytique cokéfié, dans des conditions de déshydrogénation des alcanes, pour produire une ou plusieurs oléfines, et on récupère le produit obtenu contenant une ou plusieurs oléfines à partir du catalyseur de craquage catalytique cokéfié.

2. Procédé selon la revendication 1, dans lequel le catalyseur de craquage catalytique cokéfié a une teneur en carbone dans une plage de 0,2% à 10% en poids.

3. Procédé selon la revendication 2, dans lequel le catalyseur de craquage catalytique cokéfié a une teneur en carbone dans une plage de 0,3% à 5,0% en poids.

4. Procédé selon l'une quelconque des revendications précédentes, dans lequel le catalyseur de craquage catalytique cokéfié est formé en mettant en contact un précurseur de coke avec un catalyseur de craquage catalytique.

5. Procédé selon la revendication 4, dans lequel le précurseur de coke est choisi parmi les oléfines légères, les naphtas légers et lourds, les résidus de pétrole, la boue de raffinerie, les fonds de réservoirs, les gazoils, les huiles et les fonds de recyclage FCC et les huiles de torchères.

6. Procédé selon l'une quelconque des revendications précédentes, dans lequel la charge d'alimentation est choisie parmi une ou plusieurs des matières suivantes : éthane, propane, pentane, hexane, heptane, octane, nonane, décane, isobutane, isopentanes, isohexanes, isoheptanes et isooctanes.

7. Procédé selon l'une quelconque des revendications précédentes, dans lequel la déshydrogénation de la charge d'alimentation est réalisée à une température dans une plage de 426°C à 871°C (800°F à 1600°F).

8. Procédé selon l'une quelconque des revendications précédentes, dans lequel le catalyseur de craquage catalytique comprend un composant d'oxyde cristallin à ossature tétraédrique.
